# EUROPÄISCHE PATENTSCHRIFT

(11) **EP 4 000 650 B1**
(45) Veröffentlichungstag und Bekanntmachung des Hinweises auf die Patenterteilung: **03.09.2025**
(21) Anmeldenummer: 21203789.9
(22) Anmeldetag: 20.10.2021
(51) Int. Cl.: A61L 2/10, A61L 2/26, B60N 2/00

(54) **DESINFEKTIONSVORRICHTUNG FÜR EIN KRAFTFAHRZEUG SOWIE KRAFTFAHRZEUG MIT WENIGSTENS EINER DESINFEKTIONSVORRICHTUNG**
DISINFECTION DEVICE FOR A MOTOR VEHICLE AND MOTOR VEHICLE WITH AT LEAST ONE DISINFECTION DEVICE
DISPOSITIF DE DÉSINFECTION POUR UN VÉHICULE AUTOMOBILE ET VÉHICULE AUTOMOBILE POURVU D'AU MOINS UN DISPOSITIF DE DÉSINFECTION

(30) Priorität: 18.11.2020 DE 102020214529
(43) Veröffentlichungstag der Anmeldung: 25.05.2022
(73) Patentinhaber: Volkswagen Aktiengesellschaft, 38440 Wolfsburg (DE)
(72) Erfinder: Hennemann, Sven, 30625 Hannover (DE); Weng, Michael, 10559 Berlin (DE); Ströhlein, Tobias, 38110 Braunschweig (DE)
(74) Vertreter: Bals & Vogel Patentanwälte PartGmbB

(56) Entgegenhaltungen:
- WO-A1-2016/037873
- CN-A- 111 731 171
- DE-U1- 202020 105 653
- US-A1- 2015 137 747

## Beschreibung

Die Erfindung betrifft eine Desinfektionsvorrichtung in einem Kraftfahrzeug mit den Merkmalen vom Oberbegriff des Patentanspruchs 1. Die Erfindung betrifft auch ein Kraftfahrzeug mit wenigstens einer Desinfektionsvorrichtung.

Geeigneten Möglichkeiten zur Desinfektion muss insbesondere angesichts der jüngsten Erfahrungen in der Welt angemessene Bedeutung beigemessen werden. Dass dies auch bereits für Kraftfahrzeuge erkannt wurde, zeigen beispielhaft verschiedene Dokumente, in denen Desinfektionsmöglichkeiten beziehungsweise Desinfektionsvorrichtungen in einem Kraftfahrzeug beschrieben werden.

So wird in der WO2017/204774 A1, welche den Oberbegriff der nebengeordneten Ansprüche bildet, ein Kraftfahrzeug offenbart, welches in seinem Innenraum mit mehreren Vorrichtungen ausgestattet ist, die zur Erzeugung von UV-Licht dienen. So wird unter anderem vorgeschlagen, eine UV-Lichtquelle im Aufnahmeraum einer hinteren, aufklappbaren Konsole unterzubringen. Ferner wird die Anbringung einer UV-Lichtquelle in einem Aufnahmeraum des Handschuhfachs oder in einem Ablagefach der Mittelkonsole des Kraftfahrzeugs vorgeschlagen.

In der DE 10 2015 117 559 A1 wird eine Lade- und Desinfektionsablage für ein Kraftfahrzeug beschrieben. Die Lade- und Desinfektionsablage umfasst ein drahtloses Ladegerät zum Laden eines mobilen, elektronischen Gerätes und eine Beleuchtungsvorrichtung. Konkret ist die Lade- und Desinfektionsablage in der Mittelkonsole des Kraftfahrzeugs integriert. Sie weist einen durch einen Deckel verschließbaren Aufnahmeraum auf, in den ein aufzuladendes und zu desinfizierendes, elektrisches Gerät eingelegt werden kann. Eine UV-Lichtquelle ist im Aufnahmeraum angeordnet, welcher durch ein deckelartiges Abdeckelement zum Innenraum hin abdeckbar ist.

Der DE 10 2018 002 328 A1 kann eine Vorrichtung in einem Kraftfahrzeug mit einer UV-Lichtquelle entnommen werden, welche zur Desinfektion von Fahrzeugteilen innerhalb des Fahrzeuges dient und UVC-Strahlung emittiert. Zusätzlich dient zumindest eine Lichtquelle zur Abstrahlung von für ein menschliches Auge sichtbarem Licht.

Schließlich wird in der DE 10 2012 006 972 A1 eine Reinigungseinrichtung und ein Verfahren zum Reinigen von Fahrzeuginnenräumen beschrieben. Konkret wird eine Strahlungsvorrichtung beschrieben, welche Strahlung in einem vorbestimmten Wellenlängenbereich innerhalb des UV-Lichtspektrums zwischen etwa 150 Nanometer und etwa 350 Nanometer emittiert. Die Strahlungsvorrichtung ist derart im Innenraum montiert, dass die von der Strahlungsvorrichtung emittierte Strahlung auf wenigstens einen Einrichtungsgegenstand des Fahrzeuginnenraums und/oder in den Fahrzeuginnenraum gerichtet ist.

Desinfektionsvorrichtungen sind aus den Dokumenten US 2015 137 747 A1 und DE 20 2020 105 653 U1 bekannt.

Vor dem Hintergrund des genannten Standes der Technik liegt der vorliegenden Erfindung die Aufgabe zu Grunde, eine Desinfektionsvorrichtung für ein Kraftfahrzeug vorzuschlagen, mit der die Voraussetzung für eine wirksame und gründliche Desinfektion eines Gegenstandes durch UV-Licht geschaffen wird.

Des Weiteren liegt der vorliegenden Erfindung die Aufgabe zu Grunde, ein Kraftfahrzeug mit einer Desinfektionsvorrichtung bereitzustellen.

Vorgenannte Aufgaben werden durch eine Desinfektionsvorrichtung in einem Kraftfahrzeug mit den Merkmalen von Patentanspruch 1 und durch ein Kraftfahrzeug mit den Merkmalen von Patentanspruch 11 gelöst. Vorteilhafte Ausbildungen beziehungsweise Weiterbildungen der Erfindung sind den jeweiligen Unteransprüchen entnehmbar.

Die vorliegende Erfindung geht von einer Desinfektionsvorrichtung in einem Kraftfahrzeug zur desinfizierenden Behandlung wenigstens eines Gegenstandes aus. Die Desinfektionsvorrichtung weist wenigstens einen Aufnahmeraum zur Aufnahme des zu desinfizierenden Gegenstandes auf. Es ist im Aufnahmeraum wenigstens eine Lichtquelle zur desinfizierenden Bestrahlung des Gegenstandes vorhanden.

Vorzugsweise ist die Lichtquelle zur Aussendung von Licht in einem Spektrum von etwa 100 Nanometer bis etwa 420 Nanometer, besonders bevorzugt in einem Spektrum von etwa 200 Nanometer bis etwa 280 Nanometer, und ganz besonders bevorzugt in einem Spektrum von etwa 250 Nanometer bis etwa 280 Nanometer ausgebildet. Besonders bevorzugt ist die wenigstens eine Lichtquelle also derart ausgebildet, dass sie Lichtstrahlen im UVC-Bereich abgeben kann. Es ist aber auch denkbar, dass andere Wellenlängenbereiche zur desinfizierenden Bestrahlung geeignet sind oder sich als geeignet erweisen. So ist beispielsweise auch eine desinfizierende Wirkung von abgegebenen Lichtstrahlen im Wellenlängenbereich von sichtbarem blauem Licht (Wellenlängenbereich von etwa 405 Nanometer bis etwa 415 Nanometer) vorstellbar.

Erfindungsgemäß wird nun vorgeschlagen, dass im Aufnahmeraum wenigstens eine Ablagevorrichtung in einer solchen Gebrauchsstellung vorhanden oder im Aufnahmeraum wenigstens eine Ablagevorrichtung in eine solche Gebrauchsstellung bringbar ist, durch die der Aufnahmeraum der Desinfektionsvorrichtung, in vertikaler Richtung gesehen, im Bereich der Ablagevorrichtung in einen oberen und einen unteren Aufnahmeraum unterteilt oder unterteilbar ist. Dabei ist die Ablagevorrichtung derart ausgebildet, dass sie für die von der wenigstens einen Lichtquelle abgebbaren oder abgegebenen Lichtstrahlen durchlässig oder zumindest im Wesentlichen durchlässig, jedoch zur Ablage des Gegenstandes geeignet ist.

Durch eine derartige Desinfektionsvorrichtung wird die Voraussetzung dafür geschaffen, dass eine Desinfektion eines Gegenstandes mit dem von der wenigstens einen Lichtquelle abgegebenen Licht sehr wirksam möglich ist. Insbesondere kann durch die Lage der Ablagevorrichtung im Aufnahmeraum ein auf der Ablagevorrichtung abgelegter Gegenstand auf einfache Weise von allen Richtungen mit dem von der wenigstens einen Lichtquelle abgegebenen Licht bestrahlt werden.

Gemäß der Erfindung ist die Ablagevorrichtung netz- oder gitterartig ausgebildet. Auf diese Weise kann die Ablagevorrichtung kostengünstig so ausgebildet werden, dass sie für das von der wenigstens einen Lichtquelle abgegebene Licht durchlässig oder zumindest im Wesentlichen durchlässig, jedoch zur Ablage eines Gegenstandes geeignet ist.

In einer bevorzugten Ausbildung des Erfindungsgedankens ist die Ablagevorrichtung durch eine horizontale Verschiebebewegung von einer Nichtgebrauchsstellung in eine Gebrauchsstellung oder von einer Gebrauchsstellung in eine Nichtgebrauchsstellung überführbar.

Durch diese Weiterbildung kann der Aufnahmeraum der Desinfektionsvorrichtung bei Nichtbenutzung in seiner kompletten Größe zur Ablage auch von großen Gegenständen (wie bspw. einer Flasche) genutzt werden. Ein Wechsel der Ablagevorrichtung zwischen ihrer Nichtgebrauchsstellung und einer Gebrauchsstellung kann durch ein Verschieben somit auch auf sehr einfache Weise jederzeit durchgeführt werden.

Eine höchst vorteilhafte Ausbildung des Erfindungsgedankens schlägt vor, dass die Ablagevorrichtung in ihrer Nichtgebrauchsstellung eine beutelartige Erscheinung einnimmt. Hierdurch kann die Ablagevorrichtung in ihrer Nichtgebrauchsstellung für kleine Gegenstände immer noch als platzsparende Aufbewahrungsmöglichkeit dienen.

Gemäß einer anderen Ausbildung des Erfindungsgedankens ist es aber auch denkbar, dass die Ablagevorrichtung durch eine Schwenkbewegung von einer Nichtgebrauchsstellung in ihre Gebrauchsstellung überführbar ist. Hierdurch wird ein vergleichbarer Vorteil erzielt wie bei der erstgenannten Möglichkeit einer horizontalen Verschiebebewegung der Ablagevorrichtung.

Bei beiden Alternativen ist die Ablagevorrichtung netz- oder gitterartig ausgebildet. Auch Mischformen sind denkbar.

In einer weiteren Ausbildung sind Befestigungs- oder Rastmittel vorhanden, mittels derer die wenigstens eine Ablagevorrichtung im Aufnahmeraum in einer Gebrauchsstellung fixierbar ist.

Die Befestigungs- oder Rastmittel dienen also dazu, die Ablagevorrichtung nach einer Schwenkbewegung, nach einer Verschiebebewegung oder nach einer Montagebewegung in einer Gebrauchsstellung lösbar, bevorzugt rastend, zu befestigen.

Diese Weiterbildung soll auch die Möglichkeit einschließen, dass die Ablagevorrichtung als Ganzes durch die Befestigungsmittel im Aufnahmeraum lösbar, vorzugsweise rastend, befestigbar ist. Sie kann also in dieser Ausbildung auch als Ganzes wieder vom Aufnahmeraum auf leichte Weise entfernt werden.

Eine Weiterbildung schlägt zudem vor, dass der Aufnahmeraum durch ein Abdeckelement nach außen abdeckbar oder abgedeckt ist. Das Abdeckelement kann beispielsweise deckel- oder klappenartig ausgebildet sein. Es ist auch denkbar, das Abdeckelement als ein starres Verschiebeelement oder als ein rolloartiges Element auszubilden.

Im Fall der letztgenannten Weiterbildung ist es von Vorteil, wenn die Desinfektionsvorrichtung derart ausgebildet ist, dass eine Stromzufuhr zur wenigstens einen Lichtquelle nur dann möglich ist, wenn der Aufnahmeraum durch das Abdeckelement abgedeckt oder verschlossen ist.

Eine Überprüfung, ob der Aufnahmeraum durch das Abdeckelement abgedeckt oder verschlossen ist, kann vorzugsweise elektrisch oder elektromechanisch erfolgen.

So ist in einer Weiterbildung beispielsweise denkbar, dass ein Sensor zur Erfassung einer Abdeckposition des Abdeckelementes vorhanden ist, durch den in der Abdeckposition ein Signal zur Schließung eines Stromkreises zur Stromversorgung der wenigstens einen Lichtquelle erzeugbar ist. Der Sensor kann beispielsweise als kapazitiver Näherungssensor oder als Hall-Sensor ausgebildet sein.

Um die Desinfektionsvorrichtung auch in Kraftfahrzeugen nachrüsten zu können, ist es sehr von Vorteil, wenn die Desinfektionsvorrichtung als Einsatzteil für eine Ablage in einem Kraftfahrzeug ausgebildet ist.

Um die Anzahl von Lichtquellen möglichst gering halten zu können und dennoch eine gleichmäßige Ausleuchtung eines zu desinfizierenden, auf der Ablagevorrichtung abgelegten Gegenstandes von allen Seiten zu ermöglichen, wird gemäß einer anderen vorteilhaften Ausbildung der Erfindung vorgeschlagen, dass die Desinfektionsvorrichtung oder zumindest die Oberflächen des Aufnahmeraums aus einem Material sind, welches für das von der wenigstens einen Lichtquelle abgebbare oder abgegebene Licht eine hohe Reflexionseigenschaft aufweist. Auch eine Beschichtung des Aufnahmeraums mit einem solchen Material ist vorstellbar. Es ist auch denkbar, die Oberflächen verspiegelt auszubilden.

Wie bereits erwähnt wurde, soll mit der vorliegenden Erfindung auch ein Kraftfahrzeug unter Schutz gestellt werden, welche eine erfindungsgemäße Desinfektionsvorrichtung aufweist.

Ein solches Kraftfahrzeug kann dadurch weitergebildet sein, dass die Desinfektionsvorrichtung Bestandteil einer Ablage unter einer Mittelarmlehne oder Bestandteil einer Ablage in einem Handschuhfach ist oder eine solche Ablage ausbildet.

Wenn die Desinfektionsvorrichtung Bestandteil einer Ablage unter einer Mittelarmlehne ist, so ist es sehr zweckmäßig, wenn die Mittelarmlehne gleichzeitig auch das Abdeckelement für den Aufnahmeraum ausbildet.

Bevorzugte Ausführungsbeispiele der Erfindung sind in den Figuren dargestellt und werden anhand der Figuren in der nachfolgenden Beschreibung näher erläutert. Dadurch werden auch noch weitere Vorteile der Erfindung deutlich. Gleiche Bezugszeichen, auch in unterschiedlichen Figuren, beziehen sich auf gleiche, vergleichbare oder funktional gleiche Bauteile. Dabei werden entsprechende oder vergleichbare Eigenschaften und Vorteile erreicht, auch wenn eine wiederholte Beschreibung oder Bezugnahme darauf nicht erfolgt. Die Figuren sind nicht oder zumindest nicht immer maßstabsgetreu. In manchen Figuren können Proportionen oder Abstände übertrieben dargestellt sein, um Merkmale eines Ausführungsbeispiels deutlicher hervorheben zu können.

Es zeigen, jeweils schematisch
- Fig. 1: eine perspektivische Ansicht einer Desinfektionsvorrichtung in einer ersten Ausführungsform, mit einer in Nichtgebrauchsstellung befindlichen Ablagevorrichtung,
- Fig. 2: die Desinfektionsvorrichtung gemäß Fig. 1 mit der in Gebrauchsstellung befindlichen Ablagevorrichtung,
- Fig. 3: eine Ansicht der Ablagevorrichtung von oben gemäß Ansicht III aus Fig. 2, wobei das Abdeckelement nicht mit dargestellt ist,
- Fig. 4: einen Längsschnitt einer Desinfektionsvorrichtung gemäß einer zweiten Ausführungsform,
- Fig. 5: einen Längsschnitt einer Desinfektionsvorrichtung gemäß einer dritten Ausführungsform,
- Fig. 6: eine Ansicht einer Ablagevorrichtung gemäß Ansicht VI aus Fig. 5,
- Fig. 7: einen Längsschnitt einer Desinfektionsvorrichtung gemäß einer vierten Ausführungsform,
- Fig. 8: einen Längsschnitt einer Desinfektionsvorrichtung gemäß einer fünften Ausführungsform und
- Fig. 9: ein Kraftfahrzeug mit erfindungsgemäßen Desinfektionsvorrichtungen.

In den Figuren ist mit X eine (Fahrzeug-)Längsrichtung, mit Y eine (Fahrzeug-)Querrichtung und mit Z eine (Fahrzeug-)Hochrichtung gemeint.

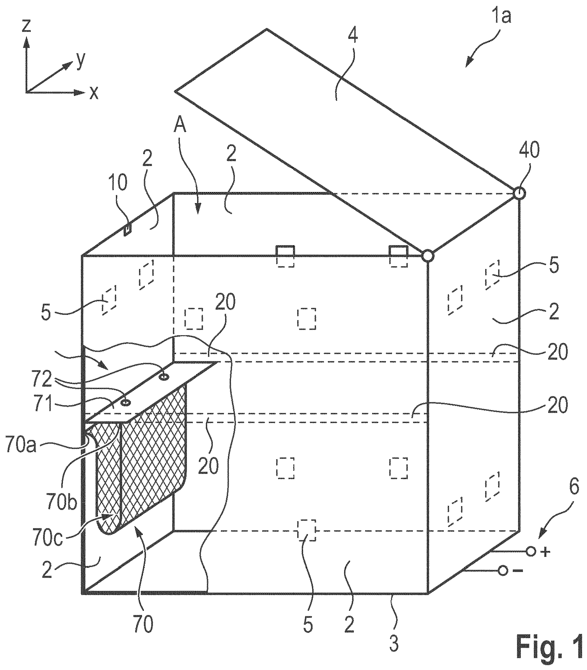

Es wird zunächst auf die Fig. 1 Bezug genommen. In dieser Figur ist eine Desinfektionsvorrichtung 1a ersichtlich. Die Desinfektionsvorrichtung 1a ist box- oder behälterartig ausgebildet und weist einen Aufnahmeraum A auf, welcher durch seitliche Wandungen 2 und einen Boden 3 gebildet wird. Der Aufnahmeraum A ist durch ein um eine Schwenkachse 40 schwenkbares Abdeckelement 4 vollständig abdeckbar oder verschließbar. An den Innenseiten der Wandungen 2 befinden sich im Aufnahmeraum A mehrere Lichtquellen 5. Die Lichtquellen 5 sind vorzugsweise derart ausgebildet, dass sie Licht in einem Wellenlängenbereich von etwa 100 bis etwa 420 Nanometer, besonders bevorzugt in einem Bereich von etwa 200 bis etwa 280 Nanometer und ganz besonders bevorzugt mit einer Wellenlänge von etwa 250 bis etwa 280 Nanometer abgeben können. Das ausgestrahlte Licht wird also besonders bevorzugt im UV-C Lichtspektrum ausgestrahlt. Eine angedeutete Stromversorgung 6 (beispielsweise das Bordnetz) dient zur elektrischen Stromversorgung der Lichtquellen 5.

An der in der Figur links ersichtlichen Wandung 2 ist eine Ablagevorrichtung 7 mit einem Netz 70 vorhanden. Insbesondere ist ein wandseitiges, erstes Ende 70a der Ablagevorrichtung 7 an der Wandung 2 befestigt. Ein gegenüberliegendes, zweites Ende 70b des Netzes 70 ist mit einem Schiebeelement 71 verbunden.

Das Schiebeelement 71 kann mit Hilfe von Handhaben 72 aus einer hier nicht näher dargestellten Arretierung gelöst und in horizontaler Richtung entlang von Führungen 20 verschoben werden, welche beispielsweise nutartig in zwei gegenüberliegenden Wandungen 2 ausgebildet sein können.

In der Figur befindet sich die Ablagevorrichtung 7 in einer Nichtgebrauchsstellung an der Wandung 2, das heißt, dass sie in dieser Position nicht zur Desinfektion eines Gegenstandes gebraucht wird. Allerdings ist ersichtlich, dass das Netz 70 in der gezeigten Nichtgebrauchsstellung der Ablagevorrichtung 7 beutelartig nach unten hängt oder mit anderen Worten eine beutelartige Erscheinung einnimmt. Lediglich zu den Seiten hin (im Bereich der Enden des Schiebeelementes 71) weist das Netz 70 einen freien Durchgang 70c auf. Somit kann die Ablagevorrichtung 7 in dieser Nichtgebrauchsstellung zumindest noch zur Aufnahme und Aufbewahrung eines kleinen Gegenstandes, wie bspw. eines Schlüssels dienen (nicht gezeigt).

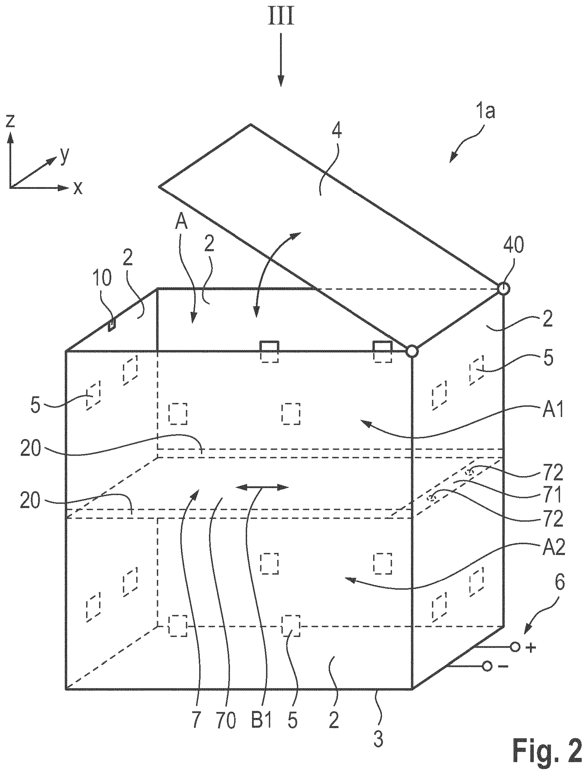

Anhand der Fig. 2 ist die Ablagevorrichtung 7 in einer Gebrauchsstellung ersichtlich, nachdem das Schiebeelement 71 bis zur gegenüberliegenden Wandung 2 in einer Verschiebebewegung B1 geschoben und dort arretiert wurde. Das Netz 70 ist somit im Aufnahmeraum A ausgebreitet und trennt den Aufnahmeraum A im Bereich der Ablagevorrichtung 7 in einen oberen Aufnahmeraum A1 und einen unteren Aufnahmeraum A2. Es sind, abweichend von der dargestellten Position durchaus mehrere Gebrauchsstellungen denkbar. Das Schiebeelement 71 kann bspw. auch nur zur Hälfte seines maximal möglichen Verschiebeweges verschoben werden.

Des Weiteren ist wenigstens ein Sensor 10 vorhanden, welcher beispielsweise als kapazitiver Näherungssensor ausgebildet sein kann und erfasst, ob und wenn das Abdeckelement 4 der Desinfektionsvorrichtung 1a den Aufnahmeraum A vollständig abdeckt oder diesen verschließt.

Nur dann wird ein Stromkreis in nicht näher dargestellter Weise geschlossen und die Stromversorgung 6 ist aktiv, so dass die Lichtquellen 5 durch Betätigung eines nicht näher dargestellten Schalters aktiviert werden können.

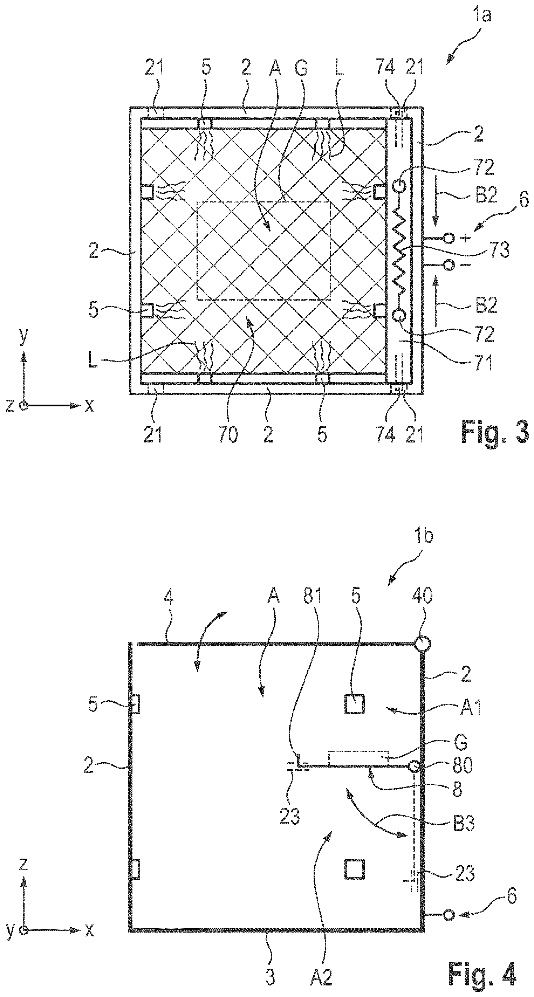

Dies ist in Fig. 3 dargestellt. Der Deckel 4, der der Übersichtlichkeit halber hier nicht dargestellt ist, deckt den Aufnahmeraum A vollständig ab. An dieser Figur ist die Struktur des Netzes 70 sehr gut erkennbar. So sind die Maschen des Netzes 70 so weit auseinander, dass von den Lichtquellen 5 abgegebenes Licht L ungehindert durch das Netz 70 hindurchtreten, jedoch ohne Weiteres einen Gegenstand G (gestrichelt angedeutet), wie beispielsweise ein Smartphone, eine Scheckkarte, ein Schlüsselbund oder dergleichen halten kann.

Ferner soll noch die Funktionsweise der Handhaben 72 näher erläutert werden. Die Handhaben 72 können beispielsweise als Vorsprünge, Mulden oder dergleichen ausgebildet sein. Sie sind mit Arretierstiften 74 bewegungsgekoppelt, welche im arretierten Zustand in Öffnungen 21 der Wandungen 2 einrasten. Zum Lösen der Rastverbindung werden die Handhaben 72 entgegen der Federkraft eines vorzugsweise als Druckfeder ausgebildeten Federelementes 73 in einer Lösebewegung B2 aufeinander zu bewegt. Dies führt dazu, dass das Schiebeelement 71 aus seiner Arretierung gelöst ist und mittels der erwähnten Verschiebebewegung B1 (vergleiche Fig. 2) in jede andere Stellung, beispielsweise in die Nichtgebrauchsstellung gemäß Fig. 1 wieder zurückgeschoben werden kann.

Anhand von Fig. 4 ist eine weitere Ausführungsform einer Desinfektionsvorrichtung 1b dargestellt. Im Unterschied zur Desinfektionsvorrichtung 1a ist hierbei eine Ablagevorrichtung 8 vorhanden, welche über eine Schwenkachse 80 schwenkbar von einer vertikalen Nichtgebrauchsstellung an der Wandung 2 (gestrichelt dargestellt) in eine horizontale Gebrauchsstellung verschwenkt werden kann (vergleiche Schwenkbewegung B3).

In beiden Stellungen kann die Ablagevorrichtung 8 mittels an den Wandungen 2 vorhandener Rastmittel 23 verrastet werden. Eine Handhabe 81 dient zum leichteren Erfassen und Bewegen der Ablagevorrichtung 8.

In diesem Ausführungsbeispiel ist die Ablagevorrichtung 8 vorzugsweise als starres, gitterartiges Element ausgebildet. Es ist aber auch denkbar, dass die Ablagevorrichtung 8 lediglich einen äußeren starren Rahmen aufweist, durch den ein Netz aufgespannt wird. Die Ablagevorrichtung 8 füllt dabei in ihrer Gebrauchsstellung nicht den gesamten Querschnitt des Aufnahmeraumes A aus.

Auch in diesem Ausführungsbeispiel wird deutlich, dass durch die Ablagevorrichtung 8 der Aufnahmeraum A im Bereich der Ablagevorrichtung 8 in einen oberen Aufnahmeraum A1 und einen unteren Aufnahmeraum A2 aufgeteilt wird. Wiederum ist hier ein auf der Ablagevorrichtung 8 aufgelegter Gegenstand G gestrichelt angedeutet.

Fig. 4 zeigt ein weiteres Ausführungsbeispiel einer Desinfektionsvorrichtung 1c. Im Unterschied zu den anderen Ausführungsformen ist hierbei eine Ablagevorrichtung 9 vorhanden, welche als Ganzes vollständig aus dem Aufnahmeraum A entfernbar ist. Bei Nichtgebrauch kann die Ablagevorrichtung 9 an einer anderen geeigneten Stelle verstaut werden, während sie bei Gebrauch in einer Montagebewegung B4 horizontal im Aufnahmeraum A mittels Rastmitteln 23 an den Wandungen 2 rastend befestigt werden kann (vergleiche gestrichelte Position 9').

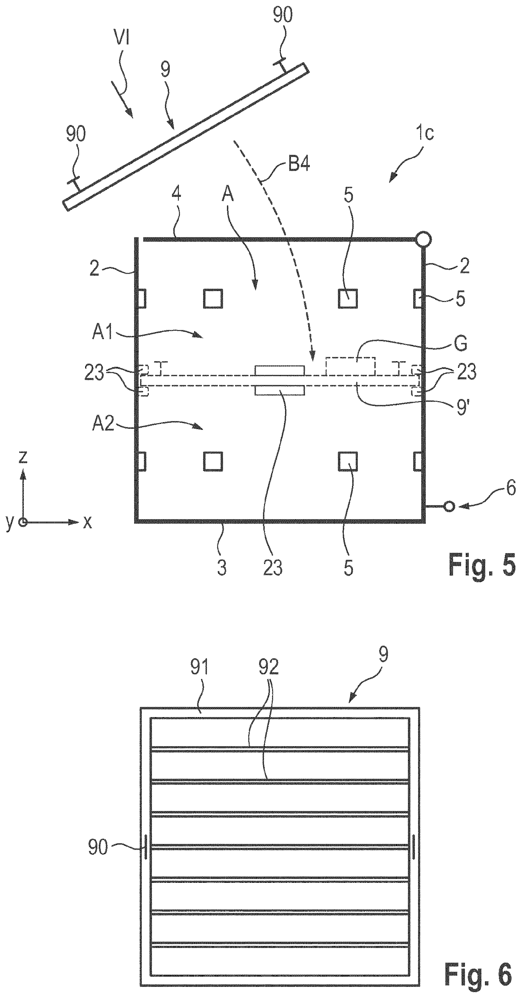

Anhand von Fig. 6 wird gut deutlich, dass die Ablagevorrichtung 9 bei diesem Ausführungsbeispiel einen äußeren Rahmen 91 aufweist, zwischen dem Stäbe 92 gehalten sind. Dadurch weist die Ablagevorrichtung 9 ein gitterartiges Aussehen auf.

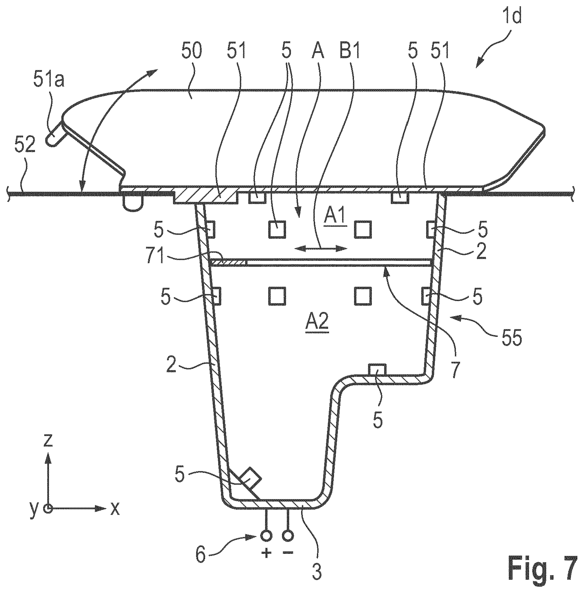

In der Fig. 7 ist nun eine Desinfektionsvorrichtung 1d ersichtlich, welche eine Ablagebox 55 unter einer Mittelarmlehne 50 ausbildet beziehungsweise Bestandteil einer solchen Ablagebox 55 ist.

Die Ablagebox 55, damit also die Desinfektionsvorrichtung 1d, weist einen Aufnahmeraum A auf, der wiederum durch eine Ablagevorrichtung 7 (vergleichbar dem ersten Ausführungsbeispiel nach Fig. 1) in einen oberen Aufnahmeraum A1 und einen unteren Aufnahmeraum A2 aufgeteilt wird. Bei der Desinfektionsvorrichtung 1d wird ein Abdeckelement des Aufnahmeraums A durch die Mittelarmlehne 50 ausgebildet, welche mitsamt einem Tragrahmen 51 mittels einer Handhabe 51a zum Öffnen beziehungsweise Abdecken des Aufnahmeraums A verschwenkbar ist (vergleiche Doppelpfeil).

In der gezeigten Figur ist die Ablagevorrichtung 7 in einer Gebrauchsstellung mit einem vollständig ausgezogenen Zustand dargestellt.

Abweichend von den bislang dargestellten Ausführungsbeispielen ist auch eine Ablagevorrichtung denkbar, welche rolloartig aus einer der Wandungen 2 der Desinfektionsvorrichtung ausgezogen werden kann.

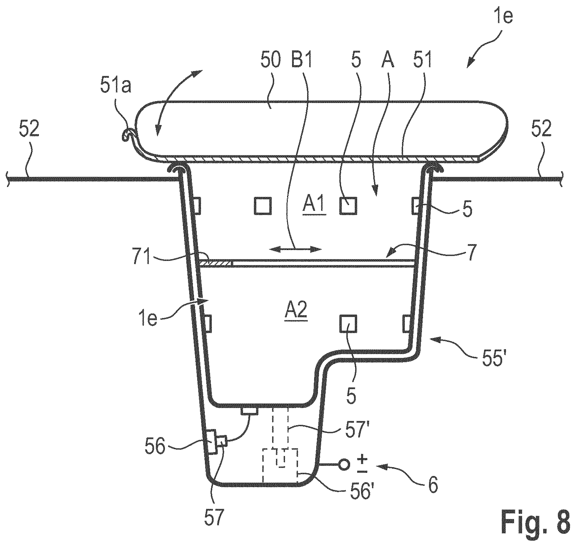

In Fig. 8 ist eine Desinfektionsvorrichtung 1e dargestellt, welche in Form eines Einsatzteils in eine Ablagebox 55' unter einer Mittelarmlehne 50 eingelassen ist.

Die Desinfektionsvorrichtung 1e weist dafür vorzugsweise eine auf die Innenkontur der Ablagebox 55' abgestimmte Außenkontur auf. Damit die Lichtquellen 5 mit Strom versorgt werden können, weist die Desinfektionsvorrichtung 1e einen USB-Stecker 57 auf, der in einen USB-Anschluss 56 der Ablagebox 55' eingesteckt werden kann.

Wiederum ist eine Ablagevorrichtung 7, wie bereits beschrieben, in vollständig ausgezogenem Zustand dargestellt, in der der Aufnahmeraum A in einen oberen Aufnahmeraum A1 und einen unteren Aufnahmeraum A2 aufgeteilt wird.

Die in der Fig. 8 dargestellte Desinfektionsvorrichtung 1e kann in dieser Weise sehr gut als Nachrüstlösung für in Fahrzeugen vorhandene Ablageboxen 55' verwendet werden.

Nach einem Hochklappen der Mittelarmlehne 50 und hergestellter Verbindung von USB-Anschluss 56 und USB-Stecker 57 kann die Desinfektionsvorrichtung 1e einfach von oben in die Ablagebox 55' eingelassen und danach die Mittelarmlehne 50 wieder abgesenkt werden.

Alternativ ist denkbar, einen USB-Anschluss und -Stecker derart auszubilden, dass eine elektrische Kontaktierung dieser Bauteile gleichzeitig mit dem vertikalen Absenken der Desinfektionsvorrichtung 1e in die Ablagebox 55' erfolgen kann (vergleiche Bezugszeichen 56' und 57').

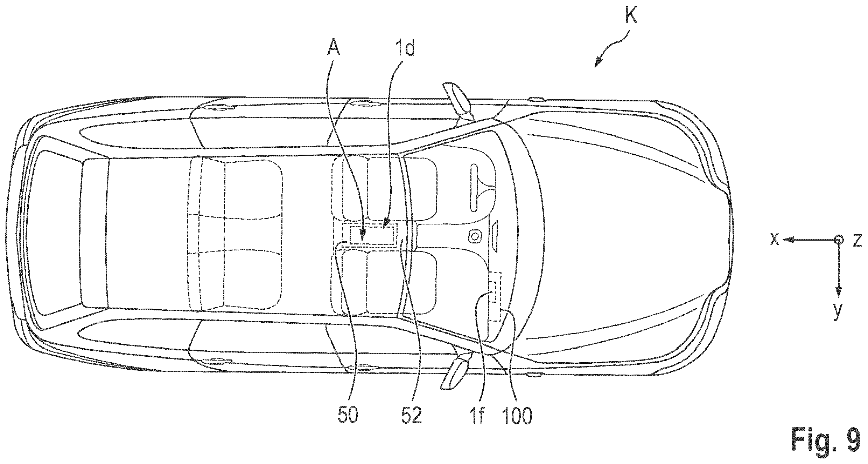

Schließlich ist anhand von Fig. 9 ein Kraftfahrzeug K dargestellt, in welchem die Desinfektionsvorrichtung 1d unter der Mittelarmlehne 50 verbaut ist (vergleiche auch Fig. 7).

Des Weiteren ist eine erfindungsgemäße Desinfektionsvorrichtung 1f innerhalb eines Handschuhfachs 100 verbaut.

Abweichend vom Ausführungsbeispiel können erfindungsgemäße Desinfektionsvorrichtungen auch noch an anderen Stellen des Kraftfahrzeugs K verbaut sein, beispielsweise im Bereich der hinteren Mittelarmlehne oder in den Tür-Seitenverkleidungen.

### Bezugszeichenliste

- 1a-1f: Desinfektionsvorrichtung
- 2: Wandungen
- 3: Boden
- 4: Abdeckelement
- 5: Lichtquellen
- 6: Stromversorgung
- 7: Ablagevorrichtung
- 8: Ablagevorrichtung
- 9: Ablagevorrichtung
- 10: Sensor
- 20: Führung
- 21: Öffnungen
- 23: Rastmittel
- 40: Schwenkachse
- 50: Mittelarmlehne
- 51: Tragrahmen
- 51a: Handhabe
- 52: Mittelkonsole
- 55, 55': Ablagebox
- 56, 56': USB-Anschluss
- 57, 57': USB-Stecker
- 70: Netz
- 70a: erstes Ende
- 70b: zweites Ende
- 70c: freier Durchgang
- 71: Schiebeelement
- 72: Handhabe
- 73: Federelement
- 74: Arretierstift
- 80: Schwenkachse
- 81: Handhabe
- 90: Handhaben
- 91: Rahmen
- 92: Stab
- 100: Handschuhfach

- A: Aufnahmeraum
- A1: oberer Aufnahmeraum
- A2: unterer Aufnahmeraum
- B1: Verschiebebewegung
- B2: Lösebewegung
- B3: Schwenkbewegung
- B4: Montagebewegung
- G: Gegenstand
- K: Kraftfahrzeug
- L: Licht

## Patentansprüche

1. Desinfektionsvorrichtung (1a, 1b, 1c, 1d, 1e, 1f) in einem Kraftfahrzeug (K) zur desinfizierenden Behandlung wenigstens eines Gegenstandes (G), mit wenigstens einem Aufnahmeraum (A) zur Aufnahme des Gegenstandes (G) und wenigstens einer Lichtquelle (5) im Aufnahmeraum (A) zur desinfizierenden Bestrahlung des Gegenstandes (G), im Aufnahmeraum (A) wenigstens eine Ablagevorrichtung (7, 8, 9) in einer solchen Gebrauchsstellung vorhanden ist, durch die der Aufnahmeraum (A), in vertikaler Richtung (Z) gesehen, im Bereich der Ablagevorrichtung (7, 8, 9) in einen oberen und einen unteren Aufnahmeraum (A1 und A2) unterteilt ist, wobei die Ablagevorrichtung (7, 8, 9) derart ausgebildet ist, dass sie für die von der wenigstens einen Lichtquelle (5) abgebbaren oder abgegebenen Lichtstrahlen (L) durchlässig, jedoch zur Ablage eines Gegenstandes (G) geeignet ist und **dadurch gekennzeichnet, dass** die Ablagevorrichtung (7, 8, 9) netz- oder gitterartig ausgebildet ist.

2. Desinfektionsvorrichtung (1a, 1d, 1e, 1f) nach Anspruch 1, **dadurch gekennzeichnet, dass** die Ablagevorrichtung (7, 8, 9) durch eine horizontale Verschiebebewegung (B1) von einer Nichtgebrauchsstellung in eine Gebrauchsstellung oder von einer Gebrauchsstellung in eine Nichtgebrauchsstellung überführbar ist.

3. Desinfektionsvorrichtung (1b) nach Anspruch 1, **dadurch gekennzeichnet, dass** die Ablagevorrichtung (8) durch eine Schwenkbewegung (B3) von einer Nichtgebrauchsstellung in ihre Gebrauchsstellung überführbar ist.

4. Desinfektionsvorrichtung (1a, 1d, 1e, 1f) nach einem der vorhergehenden Ansprüche, **dadurch gekennzeichnet, dass** die Ablagevorrichtung (7) in der Nichtgebrauchsstellung eine beutelartige Erscheinung einnimmt.

5. Desinfektionsvorrichtung (1a, 1b, 1c, 1d, 1e, 1f) nach einem der vorhergehenden Ansprüche, **dadurch gekennzeichnet, dass** Befestigungs- oder Rastmittel (23) vorhanden sind, mittels derer die wenigstens eine Ablagevorrichtung (7, 8, 9) im Aufnahmeraum (A) in ihrer Gebrauchsstellung fixierbar ist.

6. Desinfektionsvorrichtung (1a, 1b, 1c, 1d, 1e, 1f) nach einem der vorhergehenden Ansprüche, **dadurch gekennzeichnet, dass** der Aufnahmeraum (A) durch ein Abdeckelement (4) nach außen abgedeckt oder abdeckbar ist.

7. Desinfektionsvorrichtung (1a, 1b, 1c, 1d, 1e, 1f) nach Anspruch 6, **dadurch gekennzeichnet, dass** die Desinfektionsvorrichtung (1a, 1b, 1c, 1d, 1e, 1f) derart ausgebildet ist, dass eine Stromzufuhr zur Lichtquelle (5) nur dann möglich ist, wenn der Aufnahmeraum (A) durch das Abdeckelement (4) abgedeckt oder verschlossen ist.

8. Desinfektionsvorrichtung (1a, 1b, 1c, 1d, 1e, 1f) nach Anspruch 7, dass ein Sensor (10) zur Erfassung einer Abdeckposition des Abdeckelementes (4) vorhanden ist, durch den in der Abdeckposition ein Signal zur Schließung eines Stromkreises zur Stromversorgung der Lichtquelle (5) erzeugbar ist.

9. Desinfektionsvorrichtung (1e) nach einem der vorhergehenden Ansprüche, **dadurch gekennzeichnet, dass** diese als Einsatzteil für eine Ablage (55') in einem Kraftfahrzeug (K) ausgebildet ist.

10. Kraftfahrzeug (K) mit wenigstens einer Desinfektionsvorrichtung (1a, 1b, 1c, 1d, 1e, 1f) nach einem der vorhergehenden Ansprüche.

11. Kraftfahrzeug (K) nach Anspruch 10, **dadurch gekennzeichnet, dass** die Desinfektionsvorrichtung (1a, 1b, 1c, 1d, 1e, 1f) Bestandteil einer Ablage (Ablagebox 55, 55') unter einer Mittelarmlehne (50) oder einer Ablage in einem Handschuhfach (100) ist oder eine solche ausbildet.

12. Kraftfahrzeug (K) nach Anspruch 11, **dadurch gekennzeichnet, dass** die Mittelarmlehne (50) das Abdeckelement für den Aufnahmeraum (A) ausbildet.

## Claims

1. Disinfection device (1a, 1b, 1c, 1d, 1e, 1f) in a motor vehicle (K) for the disinfecting treatment of at least one object (G), comprising at least one receiving space (A) for receiving the object (G) and at least one light source (5) in the receiving space (A) for the disinfecting irradiation of the object (G), at least one storage device (7, 8, 9) being provided in the receiving space (A) in such a position of use that, as a result thereof, when viewed in the vertical direction (Z), the receiving space (A) is divided in the region of the storage device (7, 8, 9) into an upper and a lower receiving space (A1 and A2), the storage device (7, 8, 9) being designed such that it is permeable to the light rays (L) that can be emitted or are emitted by the at least one light source (5), but is suitable for storing an object (G), and **characterized in that** the storage device (7, 8, 9) is designed in the form of a net or grid.

2. Disinfection device (1a, 1d, 1e, 1f) according to claim 1,
**characterized in that** the storage device (7, 8, 9) can be transferred from a non-use position to a use position or from a use position to a non-use position by a horizontal displacement movement (B1).

3. Disinfection device (1b) according to claim 1, **characterized in that** the storage device (8) can be transferred from a non-use position into its use position by a pivoting movement (B3).

4. Disinfection device (1a, 1d, 1e, 1f) according to any of the preceding claims, **characterized in that** the storage device (7) takes on a bag-like appearance in the non-use position.

5. Disinfection device (1a, 1b, 1c, 1d, 1e, 1f) according to any of the preceding claims, **characterized in that** fastening or locking means (23) are provided, by means of which the at least one storage device (7, 8, 9) can be fixed in its position of use in the receiving space (A).

6. Disinfection device (1a, 1b, 1c, 1d, 1e, 1f) according to any of the preceding claims, **characterized in that** the receiving space (A) is covered or can be covered from the outside by a cover element (4).

7. Disinfection device (1a, 1b, 1c, 1d, 1e, 1f) according to claim 6,
**characterized in that** the disinfection device (1a, 1b, 1c, 1d, 1e, 1f) is designed such that a power supply to the light source (5) is only possible when the receiving space (A) is covered or closed by the cover element (4).

8. Disinfection device (1a, 1b, 1c, 1d, 1e, 1f) according to claim 7, that a sensor (10) for detecting a covering position of the cover element (4) is provided, by means of which a signal for closing an electrical circuit for supplying power to the light source (5) can be generated in the covering position.

9. Disinfection device (1e) according to any of the preceding claims,
**characterized in that** it is designed as an insert part for a storage compartment (55') in a motor vehicle (K).

10. Motor vehicle (K) comprising at least one disinfection device (1a, 1b, 1c, 1d, 1e, 1f) according to any of the preceding claims.

11. Motor vehicle (K) according to claim 10, **characterized in that** the disinfection device (1a, 1b, 1c, 1d, 1e, 1f) is a component of a storage compartment (storage box 55, 55') under a center armrest (50) or a storage compartment in a glove compartment (100), or forms such a compartment.

12. Motor vehicle (K) according to claim 11, **characterized in that** the center armrest (50) forms the cover element for the receiving space (A).

## Revendications

1. Dispositif de désinfection (1a, 1b, 1c, 1d, 1e, 1f) dans un véhicule automobile (K) pour le traitement désinfectant d'au moins un objet (G), comportant au moins un espace de réception (A) permettant de recevoir l'objet (G) et au moins une source lumineuse (5) dans l'espace de réception (A) pour l'irradiation désinfectante de l'objet (G), dans l'espace de réception (A) est prévu au moins un dispositif de rangement (7, 8, 9) dans une position d'utilisation telle que, avec celle-ci, l'espace de réception (A), vu dans la direction verticale (Z), est divisé dans la zone du dispositif de rangement (7, 8, 9) en un espace de rangement supérieur et en un espace de rangement inférieur (A1 et A2), dans lequel le dispositif de rangement (7, 8, 9) est conçu de telle sorte qu'il est transparent pour les rayons lumineux (L) pouvant être émis ou émis par l'au moins une source lumineuse (5), mais qu'il est approprié pour le rangement d'un objet (G), et **caractérisé en ce que** le dispositif de rangement (7, 8, 9) est conçu en forme de filet ou de grille.

2. Dispositif de désinfection (1a, 1d, 1e, 1f) selon la revendication 1,
**caractérisé en ce que** le dispositif de rangement (7, 8, 9) peut être transféré d'une position de non-utilisation à une position d'utilisation ou d'une position d'utilisation à une position de non-utilisation par un mouvement de déplacement horizontal (B1).

3. Dispositif de désinfection (1b) selon la revendication 1,
**caractérisé en ce que** le dispositif de rangement (8) peut être transféré d'une position de non-utilisation à sa position d'utilisation par un mouvement de pivotement (B3).

4. Dispositif de désinfection (1a, 1d, 1e, 1f) selon l'une des revendications précédentes, **caractérisé en ce que** le dispositif de rangement (7) prend un aspect de sac dans la position de non-utilisation.

5. Dispositif de désinfection (1a, 1b, 1c, 1d, 1e, 1f) selon l'une des revendications précédentes, **caractérisé en ce que** des moyens de fixation ou d'encliquetage (23) sont prévus, au moyen desquels l'au moins un dispositif de rangement (7, 8, 9) peut être fixé dans sa position d'utilisation dans l'espace de réception (A).

6. Dispositif de désinfection (1a, 1b, 1c, 1d, 1e, 1f) selon l'une des revendications précédentes, **caractérisé en ce que** l'espace de réception (A) est recouvert ou peut être recouvert vers l'extérieur par un élément de recouvrement (4).

7. Dispositif de désinfection (1a, 1b, 1c, 1d, 1e, 1f) selon la revendication 6,
**caractérisé en ce que** le dispositif de désinfection (1a, 1b, 1c, 1d, 1e, 1f) est conçu de telle sorte qu'une alimentation en courant vers la source lumineuse (5) n'est possible que lorsque l'espace de réception (A) est recouvert ou fermé par l'élément de recouvrement (4).

8. Dispositif de désinfection (1a, 1b, 1c, 1d, 1e, 1f) selon la revendication 7, qu'un capteur (10) permettant de détecter une position de recouvrement de l'élément de recouvrement (4) est prévu, au moyen duquel, dans la position de recouvrement, un signal peut être généré pour fermer un circuit électrique pour l'alimentation en courant de la source lumineuse (5).

9. Dispositif de désinfection (1e) selon l'une des revendications précédentes,
**caractérisé en ce que** celui-ci est conçu comme une pièce à insérer pour un rangement (55') dans un véhicule automobile (K).

10. Véhicule automobile (K) comportant au moins un dispositif de désinfection (1a, 1b, 1c, 1d, 1e, 1f) selon l'une des revendications précédentes.

11. Véhicule automobile (K) selon la revendication 10, **caractérisé en ce que** le dispositif de désinfection (1a, 1b, 1c, 1d, 1e, 1f) fait partie d'un rangement (boîte de rangement 55, 55') sous un accoudoir central (50) ou d'un rangement dans une boîte à gants (100) ou forme un tel rangement.

12. Véhicule automobile (K) selon la revendication 11, **caractérisé en ce que** l'accoudoir central (50) forme l'élément de recouvrement pour l'espace de réception (A).
